Europäisches Patentamt
European Patent Office
Office européen des brevets

Veröffentlichungsnummer: **0 275 037 A2**

# EUROPÄISCHE PATENTANMELDUNG

Anmeldenummer: 88100149.9

Anmeldetag: 08.01.88

Int. Cl.⁴: **C07C 49/84**, C07C 69/157, C07C 69/614, C07C 69/22, C07C 69/78, C08G 61/12

Priorität: **14.01.87 DE 3700809**

Veröffentlichungstag der Anmeldung: **20.07.88 Patentblatt 88/29**

Benannte Vertragsstaaten: **BE CH DE FR GB LI NL**

Anmelder: **BASF Aktiengesellschaft**
**Carl-Bosch-Strasse 38**
**D-6700 Ludwigshafen(DE)**

Erfinder: **Eggersdorfer, Manfred, Dr.**
**Hannongstrasse 18**
**D-6710 Frankenthal(DE)**
Erfinder: **Henkelmann, Jochem, Dr.**
**Ludwigshafener Strasse 44**
**D-6704 Mutterstadt(DE)**
Erfinder: **Heinz, Gerhard, Dr.**
**Im Vogelsang 2**
**D-6719 Weisenheim(DE)**
Erfinder: **Koch, Jürgen, Dr.**
**Woogstrasse 36**
**D-6708 Neuhofen(DE)**

**Aryletherketone.**

Aryletherketone der allgemeinen Formel I

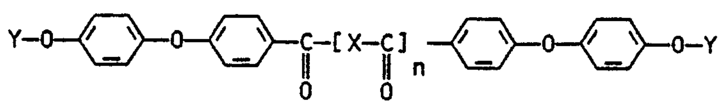

I,

in welcher
n für 0 bis 5,
X für

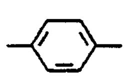 , 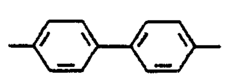 , 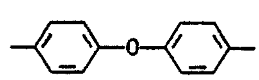

und
Y für tert.-Butyl, Acetyl, Benzyl, Benzoyl und tert.-Butoxycarbonyl sowie im Falle von n = 0 für H stehen oder deren kernsubstituierte $C_1$-$C_8$-Alkyl-, $C_1$-$C_8$-Alkoxy-, Aryl-, Chlor-oder Fluorderivate und Verwendung der Aryletherketone I zur Herstellung von hochtemperaturbeständigen Polyaryletherketonen.

EP 0 275 037 A2

## Aryletherketone

Die vorliegende Erfindung betrifft neue Aryletherketone der allgemeinen Formel I

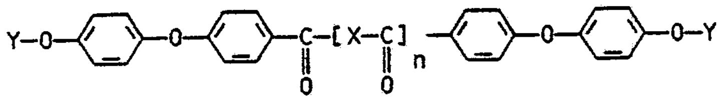 I,

in welcher
n für 0 bis 5,
X für

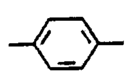 , 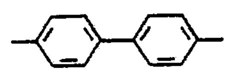 , 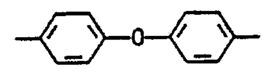

und
Y für tert.-Butyl, Acetyl, Benzyl, Benzoyl und tert.-Butoxycarbonyl sowie im Falle von n = 0 für H stehen oder deren kernsubstituierte $C_1$-$C_8$-Alkyl-, $C_1$-$C_8$-Alkoxy-, Aryl-, Chlor-oder Fluorderivate.

Weiterhin betrifft die Erfindung die Verwendung der oben definierten Verbindungen I als Monomere für die Herstellung von hochtemperaturbeständigen Polyaryletherketonen, aufgebaut aus

A) 50 bis 100 mol% wiederkehrenden Einheiten der allgemeinen Formel IV

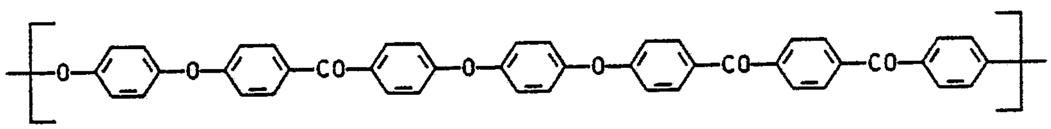 IV

oder deren kernsubstituierten $C_1$-$C_8$-Alkyl, $C_1$-$C_8$-Alkoxy, Aryl-, Chlor-oder Fluorderivaten und

B) 0 bis 50 mol% von IV verschiedenen wiederkehrenden Einheiten der allgemeinen Formeln V und/oder VI

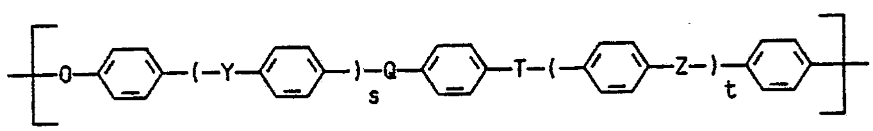 V

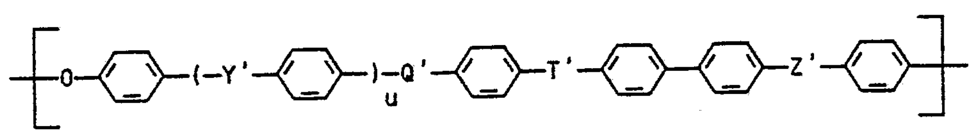 VI

oder deren kernsubstituierten $C_1$-$C_8$-Alkyl-, $C_1$-$C_8$-Alkoxy-, Aryl-, Chlor-oder Fluorderivaten wobei Q, Q', Y, Y', T, T', Z und Z' -O-, -CO-oder eine chemische Bindung sein können und mindestens einer dieser Substituenten eine -CO-Gruppe ist, und s, t und u jeweils den Wert 0 oder 1 haben.

Aus der EP-A 1879 sind Polyaryletherketone mit wiederkehrenden Einheiten der Formel

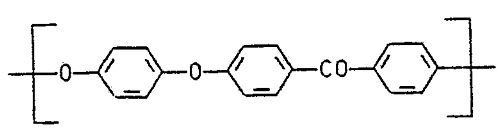

die bis zu 50 mol% andere Polyaryletherketon-Einheiten enthalten können, bekannt. Diese Produkte weisen jedoch eine für manche Anwendungszwecke nicht voll zufriedenstellende Wärmebeständigkeit auf. Hinzu kommt, daß das in dieser Anmeldung als Monomere eingesetzte 4,4'-Difluorbenzophenon sehr teuer ist.

Der Erfindung lag daher die Aufgabe zugrunde, einfach und billig zugängliche Monomere zur Verfügung zu stellen, aus denen Polyaryletherketone mit guter Wärmeformbeständigkeit hergestellt werden können.

Diese Aufgabe wird erfindungsgemäß durch die eingangs definierten Aryletherketone der Formel I gelöst. Grundsätzlich können darin die aromatischen Einheiten mit $C_1$-$C_5$-Alkyl-, $C_1$-$C_3$-Alkoxy-, Aryl-. Chlor- oder Fluorgruppen substituiert sein. Beispiele derartiger Substituenten sind Methyl, Ethyl i-Propyl, n-. i-und t-Butyl, die entsprechenden Alkoxygruppen sowie Phenoxygruppen. Im allgemeinen werden jedoch die unsubstituierten Derivate bevorzugt.

Die Herstellung der Aryletherketone I erfolgt vorteilhaft in der Weise, daß man aromatische Ketone der allgemeinen Formel II

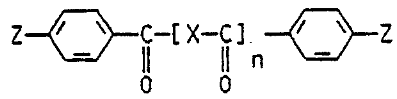

II,

in welcher Z für eine nucleophile Abgangsgruppe, z.B. für Fluor, Brom, Chlor, den Tosyl-(p-Toluolsulfonyl-) oder den Mesylrest (Methansulfonylrest) steht, mit Hydrochinon-Derivaten der allgemeinen Formel III

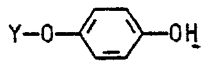

III,

in welcher Y eine der in Anspruch 1 genannten Alkoholschutzgruppen darstellt, in Gegenwart einer Base, z.B. Alkali-oder Erdalkalicarbonaten, -hydroxiden oder -alkoholaten, wie Natrium-oder Kaliumcarbonat, Natriumhydroxid, Natriumethylat, Kaliumhydroxid oder Kaliummethylat oder Alkalimetallamiden oder -hydriden wie Natriumamid, Natriumhydrid, Kaliumamid oder Kaliumhydrid in einem Verdünnungsmittel umsetzt. Zur Herstellung der hydroxylierten Verbindungen (Y = H) können die Alkoholschutzgruppen in an sich bekannter Weise, z.B. durch Behandeln mit einer Säure, z.B. Mineralsäure wie Salzsäure, gespalten werden.

Der prinzipielle Syntheseweg sei am Beispiel der Herstellung von 1,4-Bis-(p-hydroxyphenoxybenzoyl)-benzol, ausgehend von p-tert.-Butoxyphenol und 1,4-Di-(4-chlorbenzoyl)-benzol, aufgezeigt:

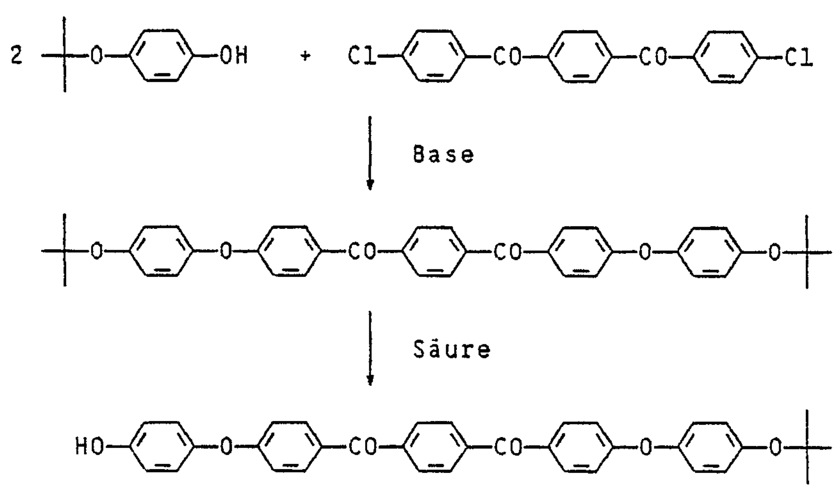


Die Ausgangsstoffe II und III sind allgemein bekannt bzw. sind nach im Prinzip bekannten Verfahren leicht zugänglich.

Die Umsetzung erfolgt in an sich bekannter Weise, so daß einige prinzipielle Anmerkungen dazu genügen. So kann man zweckmäßigerweise pro Mol des aromatischen Ketons II 2 mol des Hydrochinonderivats III sowie 0,2 bis 2,5 mol der Base verwenden. Die Umsetzung wird in Gegenwart eines inerten organischen Lösungs-oder Verdünnungsmittels durchgeführt. Hier kommen insbesondere aprotische Lösungsmittel wie z.B. N-Methylpyrrolidon, Sulfolan, Dimethylpropylenharnstoffe, Dimethylethylenharnstoff, Tetrabutylharnstoff und Hexamethylphosphorsäuretriamid in Betracht. Die Reaktionstemperatur kann breit variiert werden und liegt im allgemeinen bei 80 bis 220, insbesondere 140 bis 190°C.

Aus den so erhaltenen Produkten können die Alkoholfunktionen nach bekannten Verfahrensbedingungen, z.B. wie von A. Streitwieser, Jr., C.H. Heathcook and MacMillan in Introduction of Organic Chemistry, S. 226, Publishing Company, New York beschrieben, freigesetzt werden, so daß sich nähere Ausführungen hierzu erübrigen.

Nach der beschriebenen Umsetzungssequenz und anschließender Reinigung. z.B. durch Kristallisation, können die hydroxylierten Aryletherketone (Y = H) in guten Ausbeuten und hoher Reinheit isoliert werden. Sie eignen sich hervorragend als Monomere für Polymerisationen und Polykondensationen Vorzugsweise können sie zur Herstellung von Polyaryletherketonen, wie sie eingangs definiert sind, verwendet werden.

Die Herstellung derartiger Polyaryletherketone kann nach an sich bekannten nukleophilen oder elektrophilen Verfahren erfolgen.

Als erste Variante eines Herstellungsverfahrens ist die Polykondensation von Dihydroxyverbindungen mit Dihalogenverbindungen zu nennen, die allgemein bekannt ist.

Im vorliegenden Fall können dabei z.B. 4.4′-Di-(4-hydroxyphenoxy)-benzophenon und 1,4-Di-(4-fluorbenzoyl)-benzol oder 4,4′-Di[4-(4-hydroxyphenoxy)benzoyl]benzol und 4,4′-Dichlorbenzophenon als Monomere eingesetzt werden.

Die Verfahrensbedingungen zur Herstellung der Polyaryletherketone wie Temperatur, Druck, geeignete Lösungsmittel und evtl. Zusätze (Katalysatoren) sind die gleichen, wie sie in der EP-A 1879 beschrieben sind, so daß sich hier nähere Angaben erübrigen.

Besonders geeignet ist die Umsetzung in aprotischen polaren Lösungsmitteln in Gegenwart von wasserfreien Alkalicarbonaten als Basen. Eine besonders bevorzugte Kombination ist Diphenylsulfon als Lösungsmittel und $K_2CO_3$ als Base.

Die Menge an Diphenylsulfon beträgt im allgemeinen 5 bis 100 mol, vorzugsweise 5 bis 20 mol, bezogen auf ein Mol des Monomeren. Dies ergibt einen bevorzugten Feststoffgehalt der Reaktionslösung im Bereich von 5 bis 50 Gew.%, besonders bevorzugt 10 bis 40 Gew.%.

Das bei der Polykondensation entstehende Wasser kann mit Hilfe eines Azeotropbildners, Anlegen eines Unterdrucks oder vorzugsweise durch Einleiten eines Stickstoffstroms und Abdestillation entfernt werden.

Als Azeotropbildner eignen sich alle Verbindungen, die bei Normaldruck im Bereich der Reaktionstemperatur sieden und die sich mit dem Reaktionsgemisch homogen mischen lassen, ohne chemische Reaktionen einzugehen.

Die Reaktionstemperatur liegt im allgemeinen im Bereich von 150 bis 400°C, vorzugsweise 250 bis 400°C und insbesondere 250 bis 350°C; die Gesamt-Reaktionsdauer richtet sich nach dem gewünschten Kondensationsgrad, liegt aber im allgemeinen im Bereich von 0,1 bis 15 Stunden.

Im Anschluß an die Polykondensation können zur Stabilisierung freie Phenolat-Endgruppen mit einem Aryl-oder Alkylierungsmittel, wie z.B. Methylchlorid, umgesetzt werden. Dies erfolgt vorzugsweise bei Temperaturen von bis zu 350°C, wobei die untere Temperaturgrenze durch die Löslichkeit des Polymeren im eingesetzten Lösungsmittel abhängt.

Die Aufarbeitung der Reaktionsprodukte kann nach üblichen, an sich bekannten Verfahren erfolgen. Vorteilhaft wird aus der Schmelze ein feinteiliges Gut erzeugt, welches durch Extraktion mit einem geeigneten Lösungsmittel, z.B. Aceton, vom Lösungsmittel (z.B. Diphenylsulfon) befreit wird. Anschließend können Reste von Alkalimetallcarbonat und Alkalimetallfluorid durch Extraktion mit Wasser entfernt werden.

Die Bedingungen für die Herstellung von Polyaryletherketonen auf elektrophilem Weg einer anderen Herstellungsvariante nach der bekannten Friedel-Crafts-Acylierung, sind z.B. in der EP-A 124 276 oder EP-A 138 990 und der US-A-3 956 240 beschrieben.

In den Polyaryletherketonen der Formel IV können Einheiten der Formeln V und/oder VI, die nach grundsätzlich gleichen Verfahrensbedingungen, wie vorstehend für die Herstellung von Polyaryletherketonen mit wiederkehrenden Einheiten der Formel IV beschrieben, hergestellt werden können, statistisch verteilt oder in Form von Blöcken vorhanden sein.

Die detaillierte Beschreibung dieser Polyaryletherketone ist der zeitgleichen Anmeldung P 37 00 810.2 (O.Z 0050/38907) zu entnehmen.

Beispiele 1 bis 5

Herstellung monomerer Aryletherketone

Beispiel 1

4,4'-Di(tert.-butoxyphenoxy)benzophenon

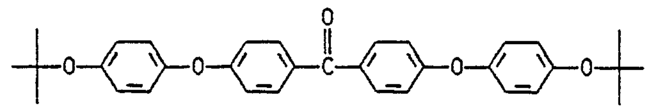

176 g (1,06 Mol) p-tert.-Butoxyphenol wurden mit 143 g (0,5 Mol) 4,4'-Dichlorbenzophenon und 76 g Kaliumcarbonat in 250 ml N-Methylpyrrolidon 1 h bei 180 bis 190°C umgesetzt. Nach Abkühlen wurde der Reaktionsansatz unter intensivem Rühren mit 100 ml $H_2O$ versetzt. Der ausgefallene Feststoff wurde abgesaugt und aus Methanol umkristallisiert.

Ausbeute: 169 g (≙ 85 % d.Th.) farblose Kristalle. Fp.: 182°C

Beispiel 2

1,4-Di-(p-tert.-butoxyphenoxybenzoyl)benzol

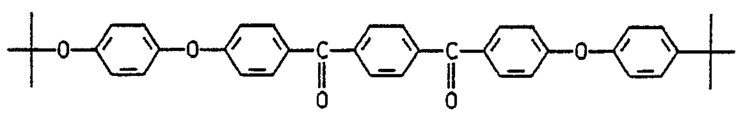

Analog Beispiel 1 wurden 166 g (1 Mol) p-tert.-Butoxyphenol mit 195 g (0,5 Mol) Di-(4-chlorbenzoyl)-benzol in 250 ml N,N'-Dimethylethylenharnstoff und 76 g Kaliumcarbonat umgesetzt und aufgearbeitet.

Ausbeute: 295 g (≙ 96 % d.Th.) farblose Kristalle. Fp.: 221°C

Beispiel 3

1,4-Di(p-hydroxy-phenoxybenzoyl)-benzol

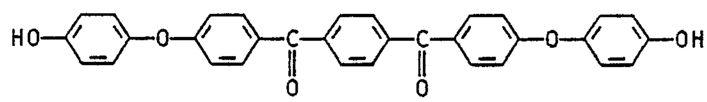

In eine Suspension von 153,5 g (0,25 Mol) 1,4-Di-(p-tert.-butoxy-phenoxybenzoyl)-benzol in 200 ml $H_2O$ wurden bei Raumtemperatur langsam 100 ml konzentrierte HCl-Lösung getropft. Anschließend rühte man 2 h bei 60° C nach. Nach Abkühlen wurde der Feststoff abgesaugt, aus Methanol umkristallisiert und getrocknet.

Ausbeute: 119 g (≙ 95 % d.Th.) farblose Kristalle. Fp.: 236°C

Beispiel 4

4,4'-Di-(p-tert.-butoxyphenoxybenzoyl)diphenyl

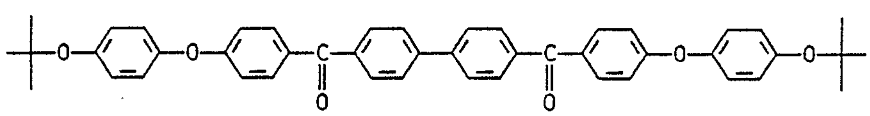

Analog Beispiel 1 wurden 166 g (1 Mol) p-tert.-Butoxyphenol mit 199 g (0,5 Mol) 4,4'-Di-(4-fluor-benzoyl)-diphenyl in 250 ml N-Methylpyrrolidon und 76 g Kaliumcarbonat umgesetzt und aufgearbeitet.

Ausbeute: 317 g (≙ 92 % d.Th.) farblose Kristalle. Fp.: 225°C

Beispiel 5

4,4'-Di-(p-hydroxyphenoxy-benzoyl)diphenyl

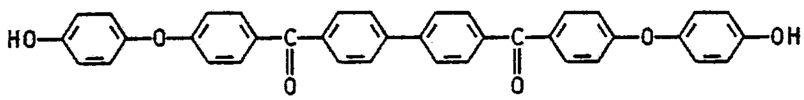

Analog Beispiel 3 wurden 172,5 g (0,25 Mol) 4,4'-Di-(p-tert.-butoxyphenoxy-benzoyl)-diphenyl in 200 ml $H_2O$ mit 100 ml konzentrierter HCl-Lösung umgesetzt und aufgearbeitet.

Ausbeute: 136 g (≙ 94 % d.Th.) farblose Kristalle. Fp.: 241 °C

Beispiele 6 und 7

Herstellung von Polyaryletherketone

Beispiel 6

79,68 g (0.2 mol) 4,4'-Di(4-hydroxyphenoxy)benzophenon, 64,46 g (0.2 mol) Bis(4-fluorbenzoyl)benzol, 30,41 g (0,22 mol) Kaliumcarbonat und 800 g Diphenylsulfon wurden in einem Vierhals-Glaskolben mit Rührer, Stickstoffeinlaß, Innenthermometer und Luftkühler auf 200 °C erhitzt und 2 Stunden bei dieser Temperatur gehalten. Dann wurde die Temperatur auf 240 °C erhöht, 1 Stunde gehalten, auf 280 °C erhöht, 1 Stunde gehalten und auf 320 °C erhöht und ebenfalls 1 Stunde bei dieser Temperatur gehalten.

Nach dem Erkalten der Reaktionsmasse wurde diese gemahlen. Das erhaltene feine Pulver wurde viermal 10 Minuten mit heißem Aceton, dreimal 10 Minuten mit kochendem Wasser und nochmals 5 Minuten mit Aceton gewaschen, um Diphenylsulfon und anorganische Bestandteile zu entfernen. Das so erhaltene Polymerpulver wurde bei 150 °C 10 Stunden im Vakuum getrocknet. Das Polymer wies nach DSC-Messungen eine Glastemperatur von 151 °C und eine Schmelztemperatur von 352 °C auf. Die reduzierte Viskosität betrug 1.41 (gemessen in konz. Schwefelsäure).

Eine nach demselben Verfahren hergestellten größere Polymermenge ließ sich problemlos und ohn Änderung des Molekulargewichts bei 400 °C extrudieren.

Beispiel 7

100,50 g (0.2 mol) 4,4'-Bis[4-(4-hydroxyphenoxy)benzoyl]benzol, 43,64 g (0.2 mol) 4,4'-Difluorbenzophenon und 30,41 g (0.22 mol) Kaliumcarbonat wurden in 800 g Diphenylsulfon unter denselben Bedingungen wie in Beispiel 6 umgesetzt.

Das so erhaltene Polymer wies eine reduzierte Viskosität von 1.38 (gemessen in konzentrierter Schwefelsäure) auf. Die physikalischen Eigenschaften sind identisch mit dem Produkt in Beispiel 6.

**Ansprüche**

1. Aryletherketone der allgemeinen Formel I

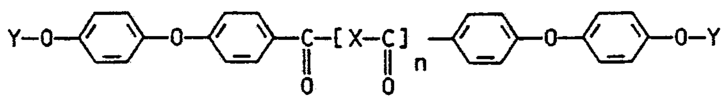

I,

in welcher
n für 0 bis 5,
X für

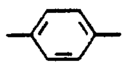 , 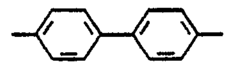 , 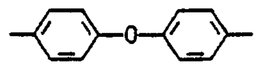

und

Y für tert.-Butyl, Acetyl, Benzyl, Benzoyl und tert.-Butoxycarbonyl sowie im Falle von n = 0 für H stehen oder deren kernsubstituierte $C_1$-$C_8$-Alkyl-, $C_1$-$C_8$-Alkoxy-, Aryl-, Chlor-oder Fluorderivate.

2. Verwendung der Aryletherketone gemäß Formel I in Anspruch 1 zur Herstellung von hochtemperaturbeständigen Polyaryletherketonen, aufgebaut aus

A) 50 bis 100 mol% wiederkehrenden Einheiten der allgemeinen Formel IV

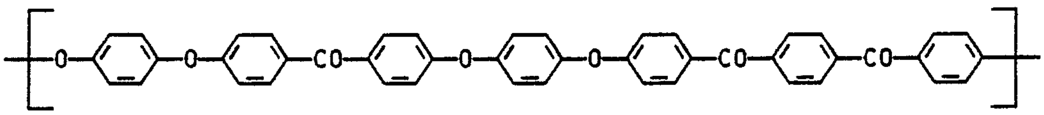 IV

oder deren kernsubstituierten $C_1$-$C_8$-Alkyl, $C_1$-$C_8$-Alkoxy, Aryl-. Chlor-oder Fluorderivaten und

B) 0 bis 50 mol% von IV verschiedenen wiederkehrenden Einheiten der allgemeinen Formeln V und/oder VI

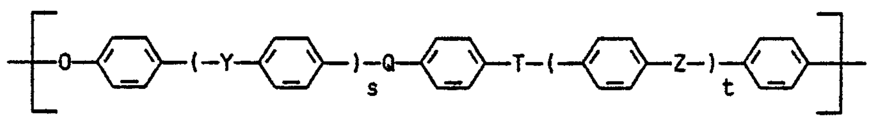 V

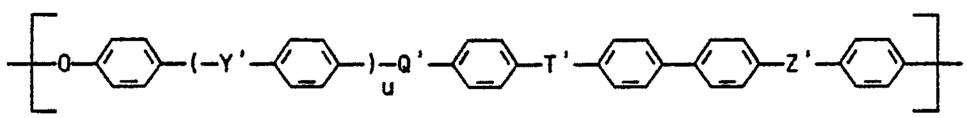 VI

oder deren kernsubstituierten $C_1$-$C_8$-Alkyl-, $C_1$-$C_8$-Alkoxy-, Aryl-, Chlor-oder Fluorderivaten wobei Q, Q', Y, Y', T, T', Z und Z' -O-, -CO-oder eine chemische Bindung sein können und mindestens einer dieser Substituenten eine -CO-Gruppe ist, und s, t und u jeweils den Wert 0 oder 1 haben.